# EUROPEAN PATENT SPECIFICATION

(11) **EP 2 750 621 B1**
(45) Date of publication and mention of the grant of the patent: **28.06.2017**
(21) Application number: 12828898.2
(22) Date of filing: 23.08.2012
(51) Int. Cl.: A61B 34/00, A61B 90/00, A61B 6/03, A61B 6/12, A61B 6/00

(54) **MICRO-ROBOT MOTION CONTROLLING SYSTEM UTILIZING DUAL SOURCE X-RAY CT EQUIPMENT**
SYSTEM ZUR MIKROROBOTERBEWEGUNGSSTEUERUNG MIT DUALQUELLEN-RÖNTEN-CT-VORRICHTUNG
SYSTÈME DE COMMANDE DE MOUVEMENT DE MICRO-ROBOT UTILISANT UN ÉQUIPEMENT DE TOMODENSITOMÉTRIE DOUBLE SOURCE

(30) Priority: 31.08.2011 KR 20110087839
(43) Date of publication of application: 09.07.2014
(73) Proprietor: Industry Foundation Of Chonnam National University, Gwangju 500-757 (KR)
(72) Inventor: PARK, Suk Ho, Gwangju 500-160 (KR); PARK, Jong Oh, Goyang-si Gyeonggi-do 411-440 (KR); JEONG, Myung Ho, Gwangju 506-304 (KR); SIM, Doo Sun, Gwangju 502-260 (KR); CHA, Kyung Rae, Gwangju 502-104 (KR); KIM, Suk Min, Gwangju 502-770 (KR); JEONG, Se Mi, Jeonju-si Jeollabuk-do 561-757 (KR); CHOI, Hyun Chol, Naju-si Jeollanam-do 520-872 (KR)
(74) Representative: Kransell & Wennborg KB
(86) International application number: PCT/KR2012/006706
(87) International publication number: WO 2013/032172

(56) References cited:
- DE-A1-102007 043 729
- KR-A- 20100 046 321
- KR-A- 20100 094 660
- KR-A- 20100 104 506
- KR-A- 20110 000 779
- KR-A- 20110 000 779
- US-A1- 2007 038 063

## Description

### Technical Field

The present invention relates, in general, to a system for controlling a position of a micro-robot using a dual source X-ray image device and, more particularly, to a system for precisely controlling a position of a micro-robot, which is electromagnetically driven, using a dual source X-ray image device.

### Background Art

Generally, an X-ray computer tomography (CT) scanner includes an X-ray source and an X-ray detector, wherein X-rays generated from the X-ray source are emitted onto and transmitted through a target object so that the X-ray detector detects the transmitted X-rays and stores measured data, which are then re-configured into images using a computer.

In the meantime, while a conventional X-ray CT scanner obtains tomography images of a target object using a single X-ray source, there is also a bi-plane X-ray image device that uses two X-ray sources [see Korean Patent No. 731052 (registered on June 15, 2007)].

A dual source X-ray image device includes two X-ray sources which are arranged perpendicular to each other in order to emit X-rays from upper and side portions of a target object, and two X-ray detectors which are positioned opposite the two X-ray sources, whereby two-dimensional images are obtained for the plane and side of the target object. The two-dimensional images are then matched with each other, obtaining three-dimensional information.

Minimally invasive operations using a micro-robot have recently been studied. In these operations, a dissection is minimized to reduce a patient's pain and shorten the convalescence period after an operation. Such a micro-robot is very small and needs the operations of its body to be precision operations so that a position of the micro-robot can be precisely controlled.

The micro-robot may be driven in an electromagnetic manner, e.g. using an electromagnetic drive, which includes Helmholtz coils, Maxwell coils and saddle coils so as to precisely drive the micro-robot (see Korean unexamined patent publication No. 10-2011-0000779 published on January 6, 2011).

In the meantime, to precisely control the position of the micro-robot, it is important to precisely detect in real-time the position of the micro-robot and a drive device to precisely drive the micro-robot so as to monitor and control the precise operation of the micro-robot along its pre-established running path.

Particularly, in order to precisely control the position of the micro-robot in the body, such as in a blood vessel where blood is flowing in a pulsatory manner, the three-dimensional position of the micro-robot must be checked in real-time and variations that occur in the external environment should affect a control signal that will drive the micro-robot.

KR 20110000779 A discloses a three-dimensional electromagnetic actuation device for driving a micro-robot comprising a main casing, a rotatable gradient saddle coil, a cylindrical casing with a gear portion and a control unit.

US 2007038063 A1 discloses a magnetic navigation device for controlling an endoscopy capsule. The magnetic navigation device comprises a coil system with a plurality of separate coils and an X-ray machine with a radiation source and a solid state radiation detector.

DE 102007043729 A1 discloses a medical system for controlling a medical instrument, such as an endoscopy capsule. The system comprises a magnetic coil system with a plurality of single coils, X-ray emitters and X-ray detectors.

### Disclosure of Invention

### Technical Problem

Accordingly, the present invention has been made keeping in mind the above problems occurring in the related art. It is intended to provide a system and method for precisely controlling a position of an electromagnetically driven micro-robot using a dual source X-ray image device.

### Solution to Problem

In an aspect, the present invention provides a system according to claim 1.

In an embodiment, the control unit may include: an image data processing module which processes image data obtained from the image pick-up unit; a matching module which obtains a coordinate and vector information of the micro-robot using image data from the processing module and a driving rotary angle of the drive unit; and a control module which controls the power supply to the coil unit to control the position of the micro-robot, using the coordinate and vector information obtained by the matching module.

In an embodiment, the image data processing module may further include a display for displaying a processed image onto a screen.

In an embodiment, the coil unit may include a pair of Helmholtz coils arranged opposite each other, and the image pick-up unit may be fixedly provided on a vertical plane between the pair of Helmholtz coils.

In an embodiment, the two X-ray sources may be arranged perpendicular to each other.

According to an example useful for understanding the invention, there is provided a method of controlling a position of an electromagnetically driven micro-robot using a dual source X-ray image device, the method including controlling the position of the micro-robot by obtaining a three-dimensional computer tomography image using X-rays from the dual X-ray sources and matching the three-dimensional image with a real-time image by adapting coordinate and vector information of the micro-robot as a feedback signal.

A coil unit for driving the micro-robot electromagnetically and the dual source X-ray image device for emitting and detecting X-rays from the two X-ray sources may be arranged perpendicular to each other and synchronously rotated to control the position of the micro-robot.

The coil unit may include a pair of Helmholtz coils arranged opposite each other, and the dual source X-ray image device may be fixedly provided on a vertical plane between the pair of Helmholtz coils.

### Advantageous Effects of the Invention

As described above, the system for controlling the position of the micro-robot can exert precision control over the micro-robot, because the coil unit that is electromagnetically driving the micro-robot and the dual source X-ray image device that is capable of tracking the position of the micro-robot are coupled together and thus are rotation-driven by a single drive unit.

Further, the method of controlling the position of the micro-robot can precisely control the micro-robot by matching the three-dimensional computer tomography image obtained using the dual source X-rays with the real-time image and using the coordinate and vector information of the micro-robot as a feedback signal.

### Brief Description of Drawings

FIG. 1 is a front view illustrating a portion of a micro-robot control system using a dual source X-ray image device according to the present invention;
FIG. 2 is a view illustrating a preferred example of a coil unit of the micro-robot control system using the dual source X-ray image device;
FIG. 3 is a perspective view illustrating a portion of the micro-robot control system using the dual source X-ray image device; and
FIG. 4 is a view illustrating the entire construction of the micro-robot control system using the dual source X-ray image device.

### * Description of Reference Signs*

100: main body 110: Image pick-up unit
120: Coil unit 121: Helmholtz coils
122: Maxwell coils 123: Saddle coils
130: Drive unit 200: Control unit
210: Image data processing module
220: Matching module 230: Control module

Hereinafter, embodiments of the present invention will be described in detail with reference to the accompanying drawings.

### Best Mode for Carrying out the Invention

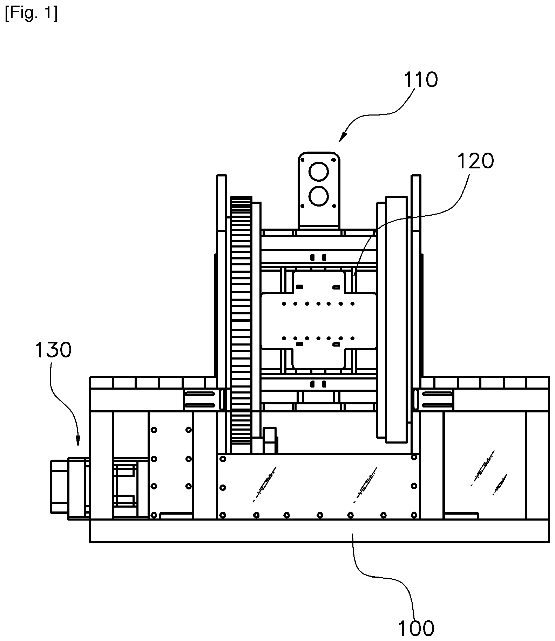

Referring to FIG. 1, a system for controlling the position of a micro-robot using a dual source X-ray image device includes a main body 100, an image pick-up unit 110 using dual X-ray sources and placed on the main body 100, a coil unit 120 for driving the micro-robot, a drive unit 130 for driving the image pick-up unit 110 and the coil unit 120 relative to the main unit, and a control unit controlling the coil unit 120 or the drive unit 130 based on image data obtained from the image pick-up unit 110 to control a position of the micro-robot.

The image pick-up unit 110, the coil unit 120, the drive unit 130, a platform on which a target object is placed, and other peripheral devices are installed on the main body 100. Although not shown, rolling wheels may be provided on a lower portion of the main body so that the main body can be easily moved to the desired position.

The image pick-up unit 110 is a unit that obtains 3-dimensional position data using bi-plane image data for a 2-dimensional plane and side face, which are obtained using dual source X-rays. The image pick-up unit may include two X-ray sources 111a and 111b arranged perpendicular to each other and two X-ray detectors 112a and 112b detecting X-rays emitted from the X-ray sources.

The X-ray detector consists of an array of sensors for detecting 2-dimensional image data.

The coil unit 120 may include a variety of coils in order to electromagnetically drive the micro-robot.

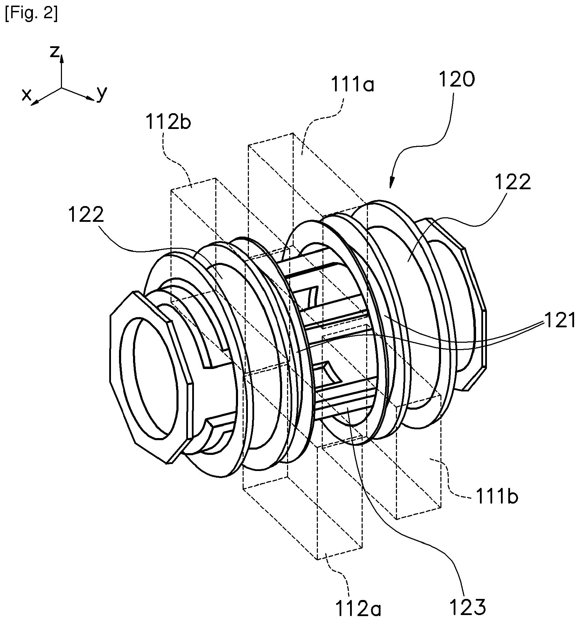

Particularly, referring to FIG. 2, the coil unit 120 may include a pair of Helmholtz coils 121 arranged opposite each other, a pair of Maxwell coils 122 coaxially positioned with the Helmholtz coils 121, and the saddle coils 123 passing through the inside of the Helmholtz coils 121 and the Maxwell coils 122.

The Helmholtz coil 121 and the saddle coils 123 are the coils that magnetizes the micro-robot and determines the forward direction, and the Maxwell coil 122 and the saddle coils 123 are the coils that interact with the magnetized force of the micro-robot according to the direction and size of the current applied thereto so as to provide thrust force to the micro-robot.

In the meantime, the construction of the coil unit is not limited to a specified form, but may adopt various types of forms. Particularly, the coil for the provision of the thrust force of the micro-robot may have diversified forms in order to provide more effective thrust force by taking into account the shapes or functions of the micro-robot.

The coil unit and the image pick-up unit may be arranged such that at least they do not interfere with each other.

Particularly, referring to FIG. 2, the image pick-up unit consisting of two X-ray sources 111a and 111b and two X-ray detectors 112a and 112b is arranged on a single plane (zy plane) such that it is fixedly arranged on a vertical plane between the pair of Helmholtz coils 121.

As previously described, coils for providing the micro-robot with thrust force and the Helmholtz coils may be arranged in an arrangement which does not hinder the image pick-up unit from obtaining image data. For example, the pair of Maxwell coils 122 may be arranged opposite each other on the axis on which the Helmholtz coils 121 are arranged, and the saddle coils 123 may pass through the inside of the Helmholtz coils 121 and the Maxwell coils 122. In this case, the image pick-up unit is fixedly arranged in a space between the Helmholtz coils and the Maxwell coils which is defined by vertical planes, such that it does not interfere with the saddle coils 123.

Further, for the position control of the micro-robot, the image pick-up unit is synchronously rotated when the coil unit is rotated.

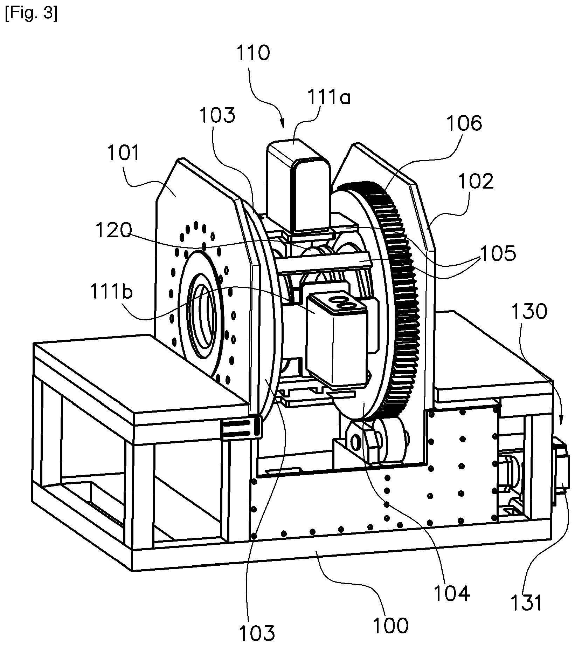

Particularly, referring to FIG. 3, on the main body 100, two stationary frames 101 and 102 are provided vertically, and first and second rotary frames 103 and 104 are coupled to each other by a plurality of fixing beams 105 such that the rotary frames are rotatable between the stationary frames 101 and 102. As previously described, the X-ray source 111a and the X-ray detector 111b of the image pick-up unit are fixed to the fixing beams 105. The coil unit 120 is fixedly installed to the first and second rotary frames 103 and 104 or the fixing beams 105, so that the coil unit rotates together with the image pick-up unit 110.

The second rotary frame 104 is provided with a slewing gear 106, which is rotated by a single drive unit 130. In an embodiment, the drive unit 130 consists of a motor 131 and a reduction gear, wherein the driving force of the motor 131 is transmitted to the slewing gear 106 via the reduction gear.

The image pick-up unit 110, the coil unit 120, and the drive unit 130, which are provided in the main body 100, are controlled by the control unit in order to precisely control the position of the micro-robot.

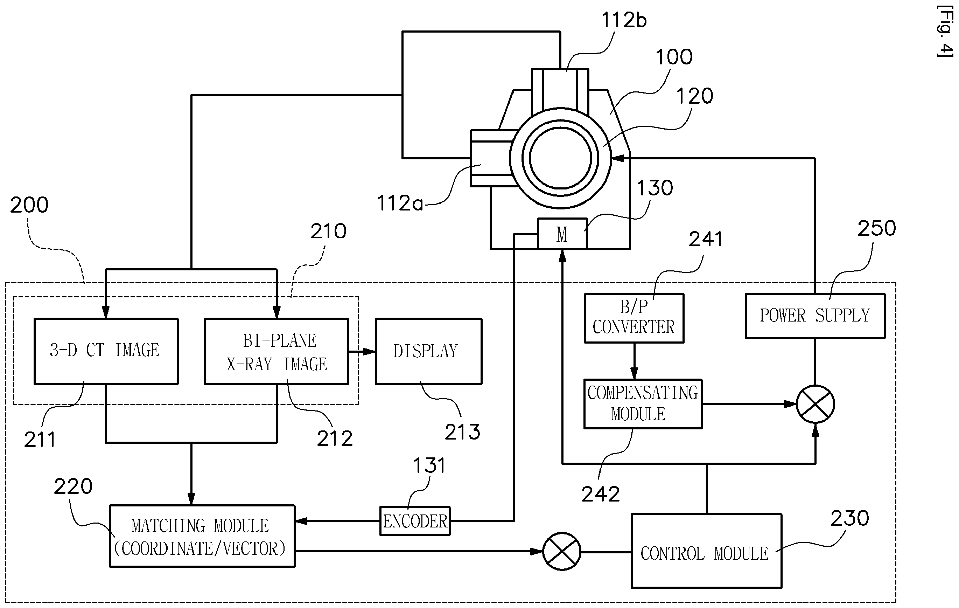

Particularly, referring to FIG. 4, the control unit 200 may include an image data processing module 210 that processes image data obtained from the image pick-up unit; a matching module 220 that obtains coordinate and vector information of the micro-robot using image data from the processing module and a driving rotary angle of the drive unit; and a control module 230 that controls the power supply to the coil unit 120 in order to control the position of the micro-robot, using the coordinate and vector
information obtained by the matching module 220.

The image data processing module 210 obtains image data 212 for two 2-dimensional planes (e.g., the xy- and yz-planes in FIG. 2) from the two X-ray detectors 112a and 112b provided in the main body 100, and then obtains a 3-dimensional computer tomography image 211 by matching using two 2-D plane image data.

In addition, the image data processing module 210 may further include a display 213 such as a monitor for outputting a real-time X-ray image or a 3-D computer tomography image to the outside.

The matching module 220 obtains the coordinate and vector information about the position of the micro-robot by going through a matching process using the rotary angle transmitted from an encoder 131, which is connected to the drive unit 130, together with the 3-D computer tomography image from the image data processing module 210 and the real-time X-ray image.

The control nodule 230 precisely controls the position of the micro-robot by rotating the drive unit 130 or controlling a power supply 250, which is connected to the coil unit 120, using the coordinate and vector information about the position of the micro-robot, which are obtained by the matching module 220, as feedback signals.

In the meantime, a BP converter 241 which detects and converts a blood pressure of a target object into an electric signal, and a compensating module 242 which receives the signal from the BP converter 241 and calculates a compensating value by analyzing the pulsatory blood flow are further provided, so that the compensating value produced by analyzing the pulsatory blood flow is applied to the control signal output from the control module 230, thereby precisely controlling the position of the micro-robot.

As described above, the system for controlling the position of the micro-robot can exert precision control over the micro-robot because of the coil unit that electromagnetically drives the micro-robot and the dual source X-ray image device which are arranged together such that they do not interfere with each other, and thus are rotation-driven by a single drive unit and because the three-dimensional computer tomography image obtained using the dual source X-rays is matched with the real-time image and then exerting real time control over the rotation of the coil unit or over the regulation of the power supply to the coil unit, using the coordinate and vector information about the position of the micro-robot as feedback signals.

In addition, the system can obtain 3-D computer tomography images using X-ray images which are obtained by rotating the image pick-up unit 110, and such 3-D computer tomography images can replace existing pre-operational images. This provides the advantages of omitting image-matching between a pre-operational image and an intra-operational image, as takes place in a conventional operation method
thereby eliminating the need for a fiducial marker to conduct matching.

Although the embodiments of the present invention have been disclosed for illustrative purposes, those skilled in the art will appreciate that various modifications, additions and substitutions are possible, without departing from the scope of the invention as disclosed in the accompanying claims.

## Claims

1. A system for controlling a position of a micro-robot, the system comprising:
a main body (100); and
a coil unit (120) provided in the main body (100) so as to generate a drive signal to electromechanically drive the micro-robot; **characterized in that** the system further comprises:
an image pick-up unit (110) provided in the main body (100), the image pick-up unit (110) includes two X-ray sources (111a, 111b) and two X-ray detectors (112a, 112b) detecting X-rays emitted from the X-ray sources (111a, 111 b);
a drive unit (130) for driving the image pick-up unit (110) and the coil unit (120), being coupled together, relative to the main body (100); and
a control unit (200) controlling the coil unit (120) or the drive unit (130) based on image data obtained from the image pick-up unit (110) to control a position of the micro-robot.

2. The system according to claim 1, wherein the control unit (200) includes:
an image data processing module (210) processing image data obtained from the image pick-up unit (110);
a matching module (220) obtaining a coordinate and vector information of the micro-robot using image data processed by the processing module (210) and a driving rotary angle of the drive unit (130); and
a control module (230) controlling a power supply to the coil unit (120) to control the position of the micro-robot, using the coordinate and vector information obtained by the matching module (220).

3. The system according to claim 2, wherein the image data processing module further includes a display (213) for displaying a processed image onto a screen.

4. The system according to claim 1, wherein the coil unit (120) includes a pair of Helmholtz coils arranged opposite each other, and the image pick-up unit (110) is fixedly provided on a vertical plane between the pair of Helm holtz coils.

5. The system according to claim 1 or 4, wherein the two X-ray sources (111a, 111 b) are arranged perpendicular to each other.

## Patentansprüche

1. System zum Steuern einer Position eines Mikroroboters, das System umfassend:
einen Grundkörper (100); und
eine Spuleneinheit (120), die so in dem Grundkörper (100) vorgesehen ist, dass sie ein Antriebssignal zum elektromechanischen Antreiben des Mikroroboters erzeugt; **dadurch gekennzeichnet, dass** das System ferner enthält:
eine Bildaufnahmeeinheit (110), die in dem Grundkörper (100) vorgesehen ist, die Bildaufnahmeeinheit (110) weist zwei Röntgenquellen (111a, 111b) und zwei Röntgendetektoren (112a, 112b) auf, die Röntgenstrahlen erkennen, die von den Röntgenquellen (111a, 111b) abgegeben werden;
eine Antriebseinheit (130) zum Antreiben der Bildaufnahmeeinheit (110) und der Spuleneinheit (120), die zusammen gekoppelt sind, relativ zu dem Grundkörper (100); und
eine Steuereinheit (200), die die Spuleneinheit (120) oder die Antriebseinheit (130) anhand von Bilddaten steuert, die aus der Bildaufnahmeeinheit (110) erhalten sind, um eine Position des Mikroroboters zu steuern.

2. System nach Anspruch 1, die Steuereinheit (200) aufweisend:
ein Bilddaten-Verarbeitungsmodul (210), das Bilddaten verarbeitet, die von der Bildaufnahmeeinheit (110) erhalten sind;
ein Abgleichmodul (220), das eine Koordinaten- und Vektorinformation des Mikroroboters mittels Bilddaten, die von dem Verarbeitungsmodul (210) verarbeitet werden, und einen Antriebsdrehwinkel der Antriebseinheit (130) erhält; und
ein Steuermodul (230), das eine Stromversorgung an die Spuleneinheit (120) mittels der Koordinaten- und Vektorinformation, die von dem Abgleichmodul (220) erhalten ist, steuert, um die Position des Mikroroboters zu steuern.

3. System nach Anspruch 2, wobei das Bilddaten-Verarbeitungsmodul ferner eine Anzeige (213) zum Anzeigen eines verarbeiteten Bilds auf einem Bildschirm aufweist.

4. System nach Anspruch 1, wobei die Spuleneinheit (120) ein Paar Helmholtz-Spulen, die einander gegenüberliegend angeordnet sind und die Bildaufnahmeeinheit (110) fest auf einer vertikalen Ebene zwischen dem Paar Helmholtz-Spulen vorgesehen ist.

5. System nach Anspruch 1 oder 4, wobei die zwei Röntgenquellen (111a, 111b) rechtwinklig zueinander angeordnet sind.

## Revendications

1. Système pour la commande d'une position d'un micro-robot, le système comprenant :
un corps principal (100) ; et
une unité de bobine (120) située dans le corps principal (100) de manière à générer un signal d'entraînement pour entraîner électro-mécaniquement le micro-robot ;
**caractérisé en ce que** le système comprend en outre :
une unité de collecte d'images (110) située dans le corps principal (100), l'unité de collecte d'images (110) comprenant deux sources de rayons X (111a, 111b) et deux détecteurs de rayons X (112a, 112b) détectant des rayons X émis à partir des sources de rayons X (111a, 111b) ;
une unité d'entraînement (130) destinée à entraîner l'unité de collecte d'images (110) et l'unité de bobine (120) accouplées ensemble, par rapport au corps principal (100) ; et
une unité de commande (200) commandant l'unité de bobine (120) ou l'unité d'entraînement (130) sur la base de données d'image obtenues à partir de l'unité de collecte d'images (110) pour commander une position du micro-robot.

2. Système selon la revendication 1, dans lequel l'unité de commande (200) comprend :
un module de traitement de données d'image (210) traitant des données d'image obtenues à partir de l'unité de collecte d'images (110) ;
un module de mise en correspondance (220) obtenant des informations de coordonnées et de vecteurs du micro-robot à l'aide de données d'image traitées par le module de traitement (210) et d'un angle de rotation d'entraînement de l'unité d'entraînement (130) ; et
un module de commande (230) commandant une alimentation électrique de l'unité de bobine (120) pour commander la position du micro-robot, à l'aide des informations de coordonnées et de vecteurs obtenues par le module de mise en correspondance (220) .

3. Système selon la revendication 2, dans lequel le module de traitement de données d'image comprend en outre un affichage (213) destiné à afficher une image traitée sur un écran.

4. Système selon la revendication 1, dans lequel l'unité de bobine (120) comprend une paire de bobines de Helmholtz disposées l'une à l'opposé de l'autre, et l'unité de collecte d'images (110) est disposée fixement sur un plan vertical entre la paire de bobines de Helmholtz.

5. Système selon la revendication 1 ou 4, dans lequel les deux sources de rayons X (111a, 111b) sont disposées perpendiculairement l'une par rapport à l'autre.
